(19) Europäisches Patentamt
European Patent Office
Office européen des brevets

(11) Veröffentlichungsnummer: **0 433 845 B1**

(12) # EUROPÄISCHE PATENTSCHRIFT

(45) Veröffentlichungstag der Patentschrift: **05.04.95**

(51) Int. Cl.$^{6}$: **C07D 473/40**

(21) Anmeldenummer: **90123792.5**

(22) Anmeldetag: **11.12.90**

(54) **Verfahren zur Herstellung von 2-Acylamino-9-acyl-6-halogen-purinen.**

(30) Priorität: **16.12.89 DE 3941658**

(43) Veröffentlichungstag der Anmeldung:
**26.06.91 Patentblatt 91/26**

(45) Bekanntmachung des Hinweises auf die Patenterteilung:
**05.04.95 Patentblatt 95/14**

(84) Benannte Vertragsstaaten:
**AT BE CH DE DK ES FR GB GR IT LI LU NL SE**

(56) Entgegenhaltungen:
**EP-A- 0 138 683**
**EP-B- 0 108 285**

(73) Patentinhaber: **HOECHST AKTIENGESELLSCHAFT**

**D-65926 Frankfurt (DE)**

(72) Erfinder: **Hertzsch, Winfried, Dr.**
**Steinauerstrasse 33**
**W-6000 Frankfurt am Main (DE)**

**EP 0 433 845 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

2-Acylamino-9-acyl-6-chlor-purine sind in der pharmazeutischen Industrie für Nucleosidsynthesen sehr vielseitig verwendbare Zwischenprodukte.

Die Herstellung von 2-Acetamino-9-acetyl-6-chlor-purin durch Acetylierung von 2-Amino-6-chlor-purin wurde bereits beschrieben (Synthetic Procedures in Nucleic Acid Chemistry, Vol. 1, 1968, W.W. Zorbach und R.S. Tipson Ed., John Wiley & Sons New York, 25-27). Dabei erfolgt die Acetylierung unter Rückflußbedingungen bevorzugt an Position 9 des Purinringes. Jedoch entsteht schon nach 5 bis 10 Minuten Reaktionszeit neben 2-Acetamino-9-acetyl-6-chlor-purin auch 2-Diacetamino-9-acetyl-6-chlor-purin als Verunreinigung. Die Gesamtausbeute der beiden Verbindungen beträgt 75 %.

Eigene Versuche zeigen darüber hinaus, daß unter den oben genannten Bedingungen bei Rückflußtemperaturen von 135 °C bis 140 °C nicht nur größere Mengen von 2-Diacetamino-9-acetyl-6-chlor-puringebildet werden, sondern auch noch ein Austausch von Chlor gegen Sauerstoff an der Position 6 des Purinringes erfolgt. Bei der Herstellung von größeren Mengen von 2-Acetamino-9-acetyl-6-chlor-purin kommen diese Nachteile besonders zum Tragen. Im Produktionsmaßstab lassen sich längere Aufheiz- und Abkühlzeiten nicht vermeiden, so daß die hohen thermischen Belastungen zu stark verunreinigten 2-Acetamino-9-acetyl-6-chlor-purin-Chargen führen.

Überraschend wurde nun gefunden, daß durch Verwendung eines Katalysators die Reinheit von 2-Acylamino-9-acyl-6-halogen-purin bei der Herstellung aus 2-Acylamino-6-halogen-purin wesentlich verbessert werden kann.

Die Erfindung betrifft somit ein Verfahren zur Herstellung der Verbindung der Formel I,

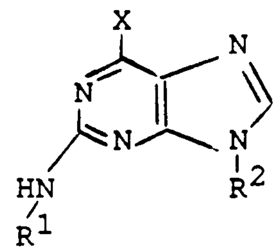

I

in der $R^1$ und $R^2$, die gleich oder verschieden sein können, für eine geradkettige oder verzweigte Alkanoylgruppe mit 2 bis 10 Kohlenstoffatomen oder eine Aralkanoylgruppe mit 7 bis 10 Kohlenstoffatomen und X für Fluor, Chlor, Brom oder Jod stehen, durch Acylierung der Verbindung der Formel II,

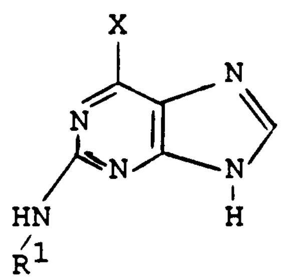

II

in der $R^1$ und X die obengenannte Bedeutung haben, das dadurch gekennzeichnet ist, daß als Katalysator Pyridin oder ein Pyridinderivat verwendet wird und die Acylierung bei einer Temperatur zwischen 10 °C und 90 °C durchgeführt wird.

Die Erfindung wird nun im folgenden detailliert beschrieben, insbesondere in ihren bevorzugten Ausführungsformen.

Unter der Bezeichnung Alkanoylgruppe mit 2 bis 10 Kohlenstoffatomen sind beispielsweise die Reste der folgenden Säuren zu verstehen:
Essigsäure, Propionsäure, n-Buttersäure, i-Buttersäure, n-Valeriansäure, i-Valeriansäure, Methylethylessigsäure, Trimethylessigsäure, Capronsäure, Caprylsäure, Caprinsäure, Heptansäure und Nonansäure. Mit der Bezeichnung Aralkanoylgruppe mit 7 bis 10 Kohlenstoffatomen sind z.B. die Reste folgender Verbindungen gemeint:
Benzoesäure, Phenylessigsäure, Phenylpropansäure, Phenyl-n-buttersäure oder Phenyl-i-buttersäure.

Die erfindungsgemäße Umsetzung von 2-Acylamino-6-halogen-purin zu 2-Acylamino-9-acyl-6-halogen-purin erfolgt in Gegenwart eines Acylierungsmittels, mit Hilfe dessen die Aminogruppe in 9-Stellung der Verbindung der Formel II mit einer geradkettigen oder verzweigten Alkanoylgruppe mit 2 bis 10 Kohlenstoffatomen oder einer Aralkanoylgruppe mit 7 bis 10 Kohlenstoffatomen substituiert werden kann. Als Acylierungsmittel können Carbonsäureanhydride verwendet werden, wie z.B. Acetanhydrid, Propionsäureanhydrid, Buttersäureanhydrid, Valeriansäureanhydrid, Caprylsäureanhydrid, Caprinsäureanhydrid oder Capronsäureanhydrid. Es können aber auch gemischte Anhydride eingesetzt werden. Ferner können als Acylierungsmittel auch Carbonsäurehalogenide, wie Acetylchlorid, Propionylchlorid, Phenylessigsäurechlorid, Phenylbutansäurechlorid, Benzoylchlorid, Acetylbromid, oder Propionylbromid verwendet werden. Die Acylierungsmittel können in einem inerten Lösungsmittel vorher gelöst werden. Als inerte Lösungsmittel können Verbindungen wie z.B. Dimethylacetamid, Toluol, Benzol oder N-Methylpyrrolidon verwendet werden.

2-Acylamino-6-halogen-purin wird mit dem Acylierungsmittel vermischt und unter Zusatz eines Katalysators erhitzt. Es können folgende Verbindungen als Katalysator verwendet werden:
Pyridin, Pyridinderivate wie z.B. 4-Dimethylaminopyridin, 4-(4-Methyl-1-piperidinyl)-pyridin, 4-Pyrrolidinopyridin, 4-Morpholinopyridin oder N-Methylimidazol. Bevorzugt wird 4-Dimethylaminopyridin verwendet.

Das molare Verhältnis von 2-Acylamino-6-halogen-purin zu dem Acylierungsmittel kann in einem Bereich von 1:1 bis 1:40 variiert werden. Bevorzugt wird in einem Bereich von 1:5 bis 1:30 gearbeitet. Das molare Verhältnis von Katalysator zu 2-Acylamino-6-halogen-purin kann in einem Bereich von 1:5 bis 1:100 schwanken. Bevorzugt wird ein molares Verhältnis von 1:35 bis 1:80 eingehalten.

Die Reaktionstemperatur liegt zwischen 10 °C und 90 °C, insbesondere zwischen 20 °C und 70 °C. Die Reaktionsdauer liegt zwischen 0,5 und 7 Stunden, insbesondere zwischen 1 und 3 Stunden. Der Reaktionsverlauf wird durch Probeentnahme und quantitative Analyse des Endproduktes mit Hilfe bekannter Titrationsmethoden ermittelt. Danach wird der Ansatz bei Temperaturen zwischen 0 °C und 10 °C noch 0,5 bis 2 Stunden gerührt. Nach Filtration erhält man die Verbindung der Formel I in hoher Reinheit.

Die Reihenfolge der einzelnen Verfahrensschritte kann auch variiert werden. Einem Fachmann fällt es nicht schwer, die optimale Reihenfolge für den Reaktionsprozeß zu finden.

Die Herstellung von 2-Acylamino-6-halogen-purinen erfolgt in an sich bekannter Weise durch Halogenierung und Acylierung von Purinen (EP 0203685; D.J. Brown, Heterocyclic Compounds, 135-197, 1971; Bowles W.A. et al., J. Med. Chem., 471-480, 6, 1963).

Im Vergleich zu dem bisher bekannten Weg zur Herstellung von 2-Acylamino-9-acyl-6-halogen-purinen eröffnet das erfindungsgemäße Verfahren den Vorteil, das Endprodukt in hoher Reinheit und Ausbeute zu erhalten. Die verwendeten Katalysatoren katalysieren die Acylierung in Position 9 des Purinringes, aber nicht die Bildung von 2-Diacylamino-9-acyl-6-halogen-purinen. Damit entfallen alle Verfahrensschritte, die zur Abtrennung der 2-Diacylamino-9-acyl-6-halogen-purin-Derivate nötig sind.

Das nachfolgend aufgeführte Beispiel dient der weiteren Erläuterung der Erfindung.

**Beispiel:**

1,1 kg (5,2 Mol) 2-Acetamino-6-chlor-purin werden in 11 l Acetanhydrid suspendiert und 87,0 g (0,71 Mol) 4-Dimethylaminopyridin hinzugefügt. Der Ansatz wird 2 Stunden bei 50 °C gerührt, anschließend abgekühlt und eine halbe Stunde bei 0 °C bis 5 °C gerührt. Das Produkt wird abfiltriert, mit Diisopropylether gewaschen und im Vakuum bei 50 °C getrocknet. Man erhält 1,16 kg 2-Acetamino-9-acetyl-6-chlor-purin.
Ausbeute: 88 % d.Th.
Reinheit. 99,1 % (Titration)

| | | |
|---|---|---|
| $^1$H-NMR (in DMSO): δ (ppm): | 2,25 | $N-\overset{\overset{O}{\|}}{C}-CH_3$ |
| | 2,93 | 9-$COCH_3$ |
| | 8,93 | 8-H |
| | 11,06 | HN-CO-C |

## Patentansprüche

1. Verfahren zur Herstellung der Verbindung der Formel I,

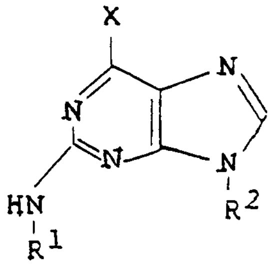

I

in der $R^1$ und $R^2$, die gleich oder verschieden sein können, für eine geradkettige oder verzweigte Alkanoylgruppe mit 2 bis 10 Kohlenstoffatomen oder eine Aralkanoylgruppe mit 7 bis 10 Kohlenstoffatomen und X für Fluor, Chlor Brom oder Jod steht, durch Acylierung der Verbindung der Formel II,

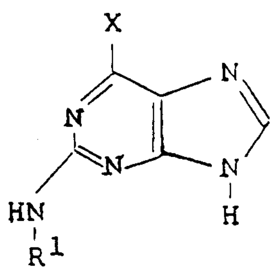

II

in der $R^1$ und X die obengenannte Bedeutung haben, das dadurch gekennzeichnet ist, daß als Katalysator Pyridin oder ein Pyridinderivat verwendet wird und die Acylierung bei einer Temperatur zwischen 10 °C und 90 °C durchgeführt wird.

2. Verfahren nach Anspruch 1, dadurch gekennzeichnet, daß als Acylierungsmittel Carbonsäureanhydrid oder Carbonsäurehalogenid verwendet werden.

3. Verfahren nach Anspruch 1, dadurch gekennzeichnet, daß als Pyridinderivat, 4-Dimethylaminopyridin, 4-(4-Methyl-1-piperidinyl)-pyridin, 4-Pyrrolidinopyridin, 4-Morpholinopyridin oder N-Methylimidazol verwendet werden.

4. Verfahren nach einem oder mehreren der Ansprüche 1 bis 3, dadurch gekennzeichnet, daß das molare Verhältnis der Verbindung der Formel II zu Acylierungsmittel im Bereich von 1:1 bis 1:40 liegt.

5. Verfahren nach Anspruch 4, dadurch gekennzeichnet, daß das molare Verhältnis im Bereich von 1:5 bis 1:30 liegt.

6. Verfahren nach einem oder mehreren der Ansprüche 1 bis 5, dadurch gekennzeichnet, daß das molare Verhältnis von Katalysator zu der Verbindung der Formel II im Bereich von 1:5 bis 1:100 liegt.

7. Verfahren nach Anspruch 6, dadurch gekennzeichnet, daß das molare Verhältnis zwischen 1:35 bis 1:80 liegt.

8. Verfahren nach Anspruch 1, dadurch gekennzeichnet, daß die Reaktionstemperatur zwischen 20 °C und 70 °C liegt.

## Claims

**1.** A process for the preparation of the compound of the formula I

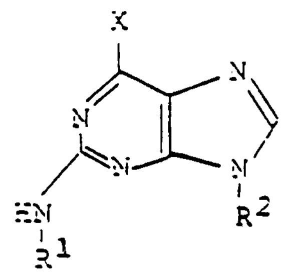

I

in which $R^1$ and $R^2$, which can be identical or different, are a straight-chain or branched alkanoyl group having 2 to 10 carbon atoms or an aralkanoyl group having 7 to 10 carbon atoms and X is fluorine, chlorine, bromine or iodine, by acylation of the compound of the formula II

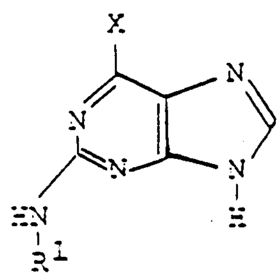

II

in which $R^1$ and X have the abovementioned meaning, using pyridine or a pyridine derivative as the catalyst and which comprises the acylation carrying out at a temperature of between 10 °C and 90 °C.

**2.** The process as claimed in claim 1, wherein carboxylic anhydride or carboxylic acid halide is used as the acylating agent.

**3.** The process as claimed in claim 1, wherein 4-dimethylaminopyridine, 4-(4-methyl-1-piperidinyl)-pyridine, 4-pyrrolidinopyridine, 4-morpholinopyridine or N-methylimidazole is used as the pyridine derivative.

**4.** The process as claimed in one or more of claims 1 to 3, wherein the molar ratio of the compound of the formula II to acylating agent is in the range from 1:1 to 1:40.

**5.** The process as claimed in claim 4, wherein the molar ratio is in the range from 1:5 to 1:30.

**6.** The process as claimed in one or more of claims 1 to 5, wherein the molar ratio of catalyst to the compound of the formula II is in the range from 1:5 to 1:100.

**7.** The process as claimed in claim 6, wherein the molar ratio is between 1:35 and 1:80.

**8.** The process as claimed in claim 1, wherein the reaction temperature is between 20 °C and 70 °C.

## Revendications

1. Procédé pour la préparation du composé de formule I

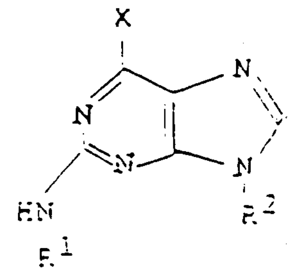

I

dans laquelle $R^1$ et $R^2$, qui peuvent être identiques ou différents, représentent un groupe alcanoyle à chaîne droite ou ramifiée ayant de 2 à 10 atomes de carbone, ou un groupe aralcanoyle ayant de 7 à 10 atomes de carbone, et X représente le fluor, le chlore, le brome ou l'iode, par acylation du composé de formule II

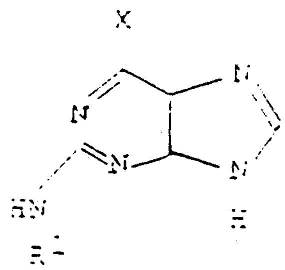

II

dans laquelle $R^1$ et X ont les significations données ci-dessus,
qui est caractérisé en ce que l'on utilise en tant que catalyseur la pyridine ou un dérivé de pyridine et on effectue l'acylation à une température comprise entre 10 et 90 °C.

2. Procédé selon la revendication 1, caractérisé en ce que l'on utilise en tant qu'agent d'acylation un anhydride ou un halogénure d'acide carboxylique.

3. Procédé selon la revendication 1, caractérisé en ce que, comme dérivé de pyridine, on utilise la 4-diméthylaminopyridine, la 4-(4-méthyl-1-pipéridinyl)-pyridine, la 4-pyrrolidinopyridine, la 4-morpholinopyridine ou le N-méthylimidazole.

4. Procède selon une ou plusieurs des revendications 1 à 3, caractérisé en ce que le rapport molaire du composé de formule II à l'agent d'acylation se situe dans la plage allant de 1:1 à 1:40.

5. Procédé selon la revendication 4, caractérisé en ce que le rapport molaire se situe dans la plage allant de 1:5 à 1:30.

6. Procédé selon une ou plusieurs des revendications 1 à 5, caractérisé en ce que le rapport molaire du catalyseur au composé de formule II se situe dans la plage allant de 1:5 à 1:100.

7. Procédé selon la revendication 6, caractérisé en ce que le rapport molaire est compris entre 1:35 et 1:80.

8. Procédé selon la revendication 1, caractérisé en ce que la température se situe entre 20 et 70 °C.